# DEMANDE DE BREVET EUROPEEN

(11) **EP 0 544 966 A1**
(43) Date de publication de la demande: **09.06.1993**
(21) Numéro de dépôt: 91500131.7
(22) Date de dépôt: 26.11.1991
(51) Int. Cl.: A61K 7/06

(54) **Lotion capillaire**

(71) Demandeur: Fernandez Martin, Juan, E-18600 Motril (Granada) (ES)
(72) Inventeur: Fernandez Martin, Juan, E-18600 Motril (Granada) (ES)
(74) Mandataire: De la Fuente Fernandez, Dionisio

(57) **Abrégé**

Procédé pour obtention d'une lotion capillaire capable d'éviter la chute des cheveux, la graisse, les pe llicules et de leur donner forme, constitué par une première phase d'eau bouillie, d'addition de persil frais hâché et l'addition postérieure d'un 9% d'alcohol à 96º et de flamber l'ensemble jusqu'à ce qu'il s'étei gne, d'ajouter postérieurement alcohol à 96º jusqu'à obtenir un mélange à 50% d'alcohol et d'eau, puis d'e refroidir, perfumer et filtrer et clarifier en ajoutant du bicarbonate de magnésium et d'un nouveau filtrage obtenant une lotion pour être employée sur les cheveux propres et secs, par des frictions légères avec les doigts, obtenant une revitalisation des cheveux, la disparition de la graisse et des pellicules et mêmê la pousse de nouveaux cheveux.

## Description

### SECTEUR POUR L'APPLICATION DE LINVENTION

Cette invention est à appliquer dans l'industrie cosmétique et similaires, ainsi que dans le secteur de la coiffure afin de fournir une lotion capable d'é viter la graisse et les pellicules, de donner de la vigueur aux cheveux et de leur fournir des caractéris tiques de protection multiple jamais obtenues jusquíci par les autres types de lotions existentes,et ayant comme base essentiellement des produits naturels, comme le persil.

### ETAT DE LA TECHNIQUE

Il existe dans l'actualité de nombreuses lotions ayant la même finalité de protection des cheveux, mais basées généralement sur des produits chimiques de diffé rentes classes, qui, si bien sont effectifs dans certains cas, leur utilisation prolongée arrive à produire un résultat négatif,en faisant varier le PH des cheveux à cause des différents ingrédients chimiques des dites lotions.

Il n'existe pas de lotions basées sur des produits naturels, sans intervention chimique comme il arrive avec le procédé auquel a trait la présente invention.

### DESCRIPTION DE L'INVENTION

Procédé pour obtention de lotion capillaire capa ble d'éviter la chute des cheveux, les pellicules, la graisse et de leur donner volume, se caractérise spéciale mentpar l'utilisation d'un nombre réduit d'ingrédients, le principal étant le persil à l'état frais , duquel on obtient un extrait, et en intervenant dans le pro cédé l 'eau et l'alcohol.

Le persil connu communément comme une plante aro matiqueemployée comme condiment dans les recettes cu linaires, est une plante qui contient une grande quantité de vitamines A et C, du fer, calcium et phosphore, et qui a en plus de tout cela la propriété de combattre cer tains toxiques du cuir chevelu, d'aider à la cicatri sation des petites blessures, de protéger des infections de prévenir les hémorragies cutanés et de réguler la graisse, maintenant en définitive la peau saine et bien nourrie.

On arrive à l'obtention du produit final avec ce procédé d'une façon simple et naturelle, sans aucune perte des propriétés, mais tout au contraire, elles augmentent et permettent postérieurement un séchage rapide, donnent de l'éclat et de la consistence aux cheveux, évitent leur chute, la graisse excessive, les pellicules, et favorisent la croissance, et même la naissance et pousse de nouveaux cheveux.

En essence le procédé consiste en l'immersion dúne certaine quantité de persil frais dans de l'eau préalablement bouillie, pour lui ajouter ensuite de l'alcohol et flamber l'ensemble jusqu'à la dispari tion de la flamme, pour ajouter ensuite de l'alcohol encore une fois pour obtenir une proportion de 50% d'alcohol et d'eau.

ON filtre et parfume avec des essences adéquates et on purifie et donne transparence par l'addition de bicarbonate de magnésium.

La lotion obtenue reste ainsi préparée pour son utilisation sur les cheveux propres et secs.

Par la suit on fera une description détaillée du procédé pour obtention d'une lotion capillaire capable d'éviter la chute des cheveux, la graisse, les pellicules, et de leur donner volume, préconisée en se référant à un exemple d'éxécution susceptible d'être varié dans tous les détails qui ne supposent aucune altération fondamentale des caractéristiques essentielles de celui-ci.

Dáprès léxemple d'éxécution cité, le procédé consis te on une série de phases consécutives; la première étant celle de bouillir dans un récipient approprié un demi litre d'eau pendant deux minutes, pour la retirer ensuite du feu et immédiatement après avoir submergé dans cette eau bouillie 75 gr de persil fris hâché, et ensuite on verse dans le récipient 45 cm³ d'alcohol à 98º.

Dans la seconde phase on procède à brûler l'alcohol versé et on laisse flamber jusqu'à la disparition de l'alcohol, lorsqu'il s'éteindra lui seul.

Dans une troisième phase, une fois l'alcohol du mé lance éteint, on procède à ajouter 205 cm³ d'alcohol à 96º pour compléter le quart de litre d'alcohol pour chaque demi litre d'eau, en laissant reposer l'ensemble jusqu'à son refroidissement, pour procéder dans une der nière phase à un filtrage et une aromatisation au moyen d 'essences et de parfums adéquats.Pour une plus grande transparence du produit on ajoute un gramme de bicarbona te de magnésium pour chaque demi litrepour la sédimenta tion des impuretés, et on procède à un nouveau filtrage et le produit reste prêt pour être utilisé.

Ce produit, utilisé une fois par jour sur les cheveux propres et secs, avec un massage energique mais léger, fourni au cuir chevelu et aux cheveux toutes les carac téristiques de vigueur,élimine les pellicules et la graisse, et la tonification générale permet que les che veux arrivent à renaître, et évite surtout la chute des cheveux par l'effet revitalisant des vitamines, du fer et du calcium, phosphore apportés par le persil frais em ployé et par l'action dégraissante et nettoyante de l'alcohol.
La forme, les matériaux et les proportions pourront va rier et en général tout ce qui soit accessoire et secon daire si l'essentialité du produit décrit n'est jamais altérée, modifiée ou changée.

## Revendications

1. Procédé pour obtention d'une lotion capillaire capa ble d'éviter la chute des cheveux, les pellicules, la graisse et de leur donner volume, se caractérise par une première phase où on fait bouillir une quantité d'eau dans un récipient, de façon que la quantité soit un 50% de la capacité de celui-ci, on maintient l'ébullition pendant deux minutes, et une fois retiré du feu on ajoute un 15% de la quantité d'eau, de persil frais hâché, et on submerge totalement le persil sous l'eau, pour ajouter ensuite un 9% d'alcohol à 96%, et on procède à le faire flamber jusqu'à ce qu'il s'éteigne lui seul, et à ce mo me nt on ajoute l'alcohol en quantité adéquate pour que lénsemble reste avec le 50%.

2. Procédé pour obtention d'une lotion capillaire capa ble d'éviter la chute des cheveux, les pellicules et la graisse et de leur donner volume, d'après la revendica tion pre mière, se caractérise par une dernière phase où l'on procde, une fois le produit refroidi, au filtrage et à láromatisation de celui-ci avec des essences pro pres à cet usage, et à une clarification par l'addition d'un gramme de bicarbonate de magnésium pour chaque demi litre, pour la sédimentation et un postérieur deuxième filtrage, la lotion restant apte pour son utilisation. Tel qu'il est décrit et revendiqué dans la présente Mémoire descriptive formée par cinq feuilles numérotées et dactylographiées sur une seule face.
